# EUROPÄISCHE PATENTSCHRIFT

(11) **EP 1 735 265 B1**
(45) Veröffentlichungstag und Bekanntmachung des Hinweises auf die Patenterteilung: **18.03.2009**
(21) Anmeldenummer: 04804094.3
(22) Anmeldetag: 20.12.2004
(51) Int. Cl.: C07C 209/48, C07C 211/12, C07C 255/24

(54) **NITRILHYDRIERUNG IN GEGENWART IONISCHER FLÜSSIGKEITEN AN HETEROGENEN KATALYSATOREN**
NITRILE HYDROGENATION ON HETEROGENEOUS CATALYSTS IN THE PRESENCE OF IONIC LIQUIDS
HYDROGENATION DE NITRILE EN PRESENCE DE LIQUIDES IONIQUES SUR DES CATALYSEURS HETEROGENES

(30) Priorität: 22.12.2003 DE 10361071
(43) Veröffentlichungstag der Anmeldung: 27.12.2006
(73) Patentinhaber: BASF SE, 67056 Ludwigshafen (DE)
(72) Erfinder: WEISKOPF, Verena, 67577 Alsheim (DE); GERLACH, Till, 67071 Ludwigshafen (DE); WENZ, Kirsten, 68161 Mannheim (DE)
(74) Vertreter: Isenbruck, Günter
(86) Internationale Anmeldenummer: PCT/EP2004/014495
(87) Internationale Veröffentlichungsnummer: WO 2005/061429

(56) Entgegenhaltungen:
- WO-A-02/096862
- US-A- 3 919 271
- WILLIAM T. MILLER ET.AL.: "SUBSTITUTION AND ADDITION REACTIONS OF THE FLUOROOLEFINS. IV. REACTIONS OF FLUORIDE ION WITH FLUOROOLEFINS" J. AM. CHEM. SOC., Bd. 82, 1960, Seiten 3091-3099, XP002326736

## Beschreibung

Die vorliegende Erfindung betrifft ein Verfahren zur Hydrierung von in organischen Verbindungen enthaltenen Nitrilfunktionen an mindestens einem heterogenen Katalysator.

Nitrile, Dinitrile oder Trinitrile sind weit verbreitete Edukte in der chemischen, pharmazeutischen und agrochemischen Industrie. Durch Hydrierung von Nitrilen bzw. Dinitrilen können Amine, Aminonitrile bzw. Diamine erhalten werden, die als Grundstoffe oder Additive für Polymere, als oberflächenaktive Substanz, Chelatbildner oder allgemein als Intermediate in der chemischen Synthese verwendet werden. WO-A-02/096862 offenbart z.B. die Hydrierung von Adipodinitril zu Hexamethylenediamin.

Zur Hydrierung von Nitrilfunktionen eignen sich homogene und heterogene Verfahren. Industriell von Vorteil sind dabei heterogene Verfahren, da die Verwendung von heterogenen Katalysatoren und die Rückführung dieser Katalysatoren in der Regel wesentlich einfacher und billiger ist als bei homogenen Katalysatoren.

Zur heterogenen Hydrierung von Nitrilen bzw. Dinitrilen werden Katalysatoren verwendet, die beispielsweise eines oder mehrere der Metalle Nickel, Kobalt, Kupfer, Palladium, Platin, Rhenium, Ruthenium und Eisen enthalten. Diese Katalysatoren zur Hydrierung von Nitrilen bzw. Dinitrilen weisen im Allgemeinen eine unzureichende Standzeit auf. Darüber hinaus ist die Selektivität bei Hydrierungen won Nitrilen zu primären, sekundären oder tertiären Aminen unzureichend: So werden beispielsweise, wenn die Herstellung von primären Aminen gewünscht ist, häufig auch die nicht erwünschten, höher substituierten Amine gebildet. Bei der Hydrierung von Dinitrilen ist es darüber hinaus häufig nicht möglich, das intermediäre Aminonitril bei hohen Umsätzen des Dinitrils und gleichzeitig hohen Selektivitäten zu erhalten.

Höhere Standzeiten und verbesserte Selektivitäten bei der Hydrierung von Nitrilen können dadurch erreicht werden, dass die Hydrierung bei hohem Druck und hoher Temperatur in Gegenwart von überschüssigem Ammoniak durchgeführt wird. Darüber hinaus "wird häufig ein Lösemittel verwendet, das entweder aus einem der Reaktionspartner (z.B. Nitril oder Amin) oder aus gängigen Lösemitteln (z.B. organische Lösemittel oder Wasser) besteht.

Werden Ammoniak oder konventionelle Lösemittel in den bekannten Verfahren zur Hydrierung von Nitrilen verwendet, um die zuvor erwähnten Nachteile zu vermeiden, ist im Allgemeinen neben einem erhöhten Druck während der Reaktion eine aufwendige Aufarbeitung zur Rückführung des Ammoniaks oder des konventionellen Lösemittels erforderlich.

Eine weitere Möglichkeit, die Selektivität in Verfahren zur Hydrierung von Nitrilen zu verbessern, ist die Verwendung von Additiven. Als Additive werden neben Alkalimetallhydroxiden oder ähnlichem mit oder ohne Wasser beispielsweise Tetraalkylammonium- bzw. Tetraalkylphosphoniumsalze, die Hydroxide, Azide, Fluoride, Thiocyanide oder Cyanate als Gegenionen besitzen, verwendet. Die Salze, die unter den gegebenen Reaktionsbedingungen der heterogenen Hydrierung von Nitrilen als Feststoff oder in Lösung anfallen, werden in den bekannten Verfahren im Allgemeinen nicht recycliert, sondern müssen umweltgerecht entsorgt werden und verteuern somit die Verfahrensweise.

US 3,919,271 beschreibt die Hydrierung von Nitrilen in Gegenwart von Dispersionen eines Metallhalogenids in Ammonium- oder Phosphoniumstannaten oder -germanaten. Die nur in katalytischen Mengen eingesetzten Dispersionen werden dabei nicht zurückgewonnen. Durch die Verwendung dieser Salze, die einen Schmelzpunkt oberhalb von 100°C aufweisen, werden allerdings die Möglichkeiten der Temperaturführung und Steuerung der Polaritäten der zu hydrierenden Mischung eingeschränkt. Zudem sind Stannate bzw. Germanate aufgrund ihrer Toxizität und problematischen Abfallbeseitigung im industriellen Maßstab unerwünscht.

Eine Selektivitätserhöhung in der Nitrilhydrierung kann auch durch Säurezugabe erzielt werden. Hierdurch kann, neben einer eventuellen positiven Veränderung aktiver Zentren des Katalysators, eine Abtrennung des gebildeten Amins über das Ammoniumsalz erzielt werden. Problematisch ist aber, dass ein Salz anfällt, aus dem das Amin mit Laugen freigesetzt werden muss, so dass durch die notwendige Feststoffhandhabung diese Verfahrensweise verteuert wird.

Aufgabe der vorliegenden Erfindung ist es somit, ein Verfahren zur Hydrierung von Nitrilfunktionen an mindestens einem heterogenen Katalysator bereitzustellen, welches die zuvor beschriebenen Nachteile im Wesentlichen vermeidet. Dabei soll vorzugsweise zum Erreichen gleicher oder verbesserter Selektivität und Standzeit ein geringeres Molverhältnis von Ammoniak zu Nitril als in den bekannten Verfahren erforderlich sein.

Besonders bevorzugt soll das Verfahren ermöglichen, auf die Verwendung von Ammoniak vollständig zu verzichten. Eine weitere Aufgabe der vorliegenden Erfindung besteht darin, ein Verfahren zur Hydrierung von Nitrilfunktionen an mindestens einem heterogenen Katalysator bereitzustellen, bei dem der für eine ausreichende Selektivität und Standzeit notwendige Gesamtdruck im Vergleich zu den bekannten Verfahren vorzugsweise reduziert ist. Dabei sollen die zuvor genannten Aufgaben unter möglichst geringem Anfall von Abfall und einer einfachen und daher wirtschaftlichen Aufarbeitung der verschiedenen Produkte bzw. Rückführungen erfolgen.

Die Lösung der Aufgabe geht aus von einem Verfahren zur Hydrierung von in organischen Verbindungen enthaltenen Nitrilfunktionen an mindestens einem heterogenen Katalysator. Das erfindungsgemäße Verfahren ist dann dadurch gekennzeichnet, dass die Hydrierung in Gegenwart einer ionischen Flüssigkeit durchgeführt wird.

Die ionische Flüssigkeit kann dabei als Lösemittel fungieren.

So ist es beispielsweise möglich, in dem erfindungsgemäßen Verfahren die ionische Flüssigkeit als ausschließliches Lösemittel, d.h. in einem großen Überschuss zu Edukt und Produkt, zu verwenden.

Alternativ ist es aber auch möglich, die ionische Flüssigkeit in Kombination mit einem anderen, konventionellen Lösemittel zu verwenden. Dafür bietet sich jedes Verhältnis zwischen ionischer Flüssigkeit und Lösemittel an. Besonders bevorzugt sind allerdings Verhältnisse mit 1 bis 99 Vol.%, insbesondere 1 bis 50 Vol.%, speziell 1 bis 25 Vol.%, ionischer Flüssigkeit in dem konventionellen Lösemittel, jeweils bezogen auf die Gesamtreaktionsmischung.

Das konventionelle Lösemittel kann dabei dieselbe, eine ähnliche oder eine komplementäre Polarität zur ionischen Flüssigkeit aufweisen. Darüber hinaus kann das konventionelle Lösemittel mit der ionischen Flüssigkeit mischbar oder nicht-mischbar sein bzw. mit der ionischen Flüssigkeit stabile oder nicht-stabile Emulsionen bilden.

Als konventionelles Lösemittel kann auch das Edukt, d.h. das zu hydrierende Nitril, und/oder das Produkt, d.h. das herzustellende Amin bzw. Aminonitril, ohne oder mit zusätzlichem konventionellem Lösemittel verwendet werden.

Als konventionelle Lösemittel geeignet sind polare Lösemittel, die ausgewählt sind aus der Gruppe, bestehend aus Methanol, Ethanol, höhere Alkohole, Polyole, Pyridin, Chinolin, Dichlormethan, Chloroform, Alkylnitrile wie Acetonitril, Pentennitril-Isomeren, Adipodinitril, Dimethylformamid, Dimethylsulfoxid, Wasser, Aceton, höhere Ketone, Tetrahydrofuran, 1,4-Dioxan, Vinylpyrrolidon, N-Methylpyrrolidon, Estern, beispielsweise Essigester, Säuren, beispielsweise Essigsäure, Propionsäure oder Adipinsäure, Aminen bzw. Diamine, beispielsweise Hexamethylendiamin, Aminocapronitril, Trialkylamine und kurzkettige Ether, beispielsweise Diethylether. Geeignete unpolare Lösemittel werden vorzugsweise ausgewählt aus der Gruppe, bestehend aus Kohlenwasserstoffen, Aromaten, beispielsweise Toluol, Xylol, Mesitylen und oligo- bzw. polymeren Ethern.

Darüber hinaus ist es auch denkbar, dass die ionische Flüssigkeit als Additiv in dem erfindungsgemäßen Verfahren zur Hydrierung von Nitrilen verwendet wird. Dabei beträgt der Gehalt an ionischer Flüssigkeit vorzugsweise 0,0001 bis 10 Vol.%, besonders bevorzugt 0,001 bis 5 Vol.%, jeweils bezogen auf die Gesamtreaktionsmischung.

In dem erfindungsgemäßen Verfahren ist es bevorzugt, dass die ionische Flüssigkeit bei der Hydrierung im flüssigen Zustand vorliegt. Damit entfällt eine teure und aufwendige Feststoffhandhabung.

Die Mengenverhältnisse von zu hydrierendem Nitril bzw. Dinitril und ionischer Flüssigkeit und somit die genaue Zusammensetzung des Reaktionsgemisches ist abhängig vom Nitril bzw. entstehenden Amin und den gewählten Reaktionsbedingungen. Die Mengenverhältnisse werden vorzugsweise so eingestellt, dass sowohl eine möglichst hohe Ausbeute als auch eine möglichst hohe Selektivität erzielt werden. Dafür muss die Menge des eingebrachten Wasserstoffs ausreichend sein, um zumindest einen Teil des Nitrils zu hydrieren. Die genauen Druck- und Temperaturverhältnisse richten sich dabei nach den verwendeten Reaktionspartnern bzw. den gewählten Reaktionsbedingungen.

### Ionische Flüssigkeit.

Ionische Flüssigkeiten sind nach der Definition von Wasserscheid und Keim in "Angewandte Chemie" 2000, 112, Seiten 3926-3945 bei relativ niedrigen Temperaturen schmelzende Salze mit nicht-molekularem, sondern ionischem Charakter. Sie sind bereits bei relativ niedrigen Temperaturen flüssig und dabei relativ niedrig viskos. Sie besitzen sehr gute Löslichkeiten für eine große Anzahl organischer, anorganischer und polymerer Substanzen. Darüber hinaus sind sie im Allgemeinen nicht brennbar, und haben keinen messbaren Dampfdruck.

Ionische Flüssigkeiten werden aus positiven und negativen Ionen gebildet, sind jedoch insgesamt ladungsneutral. Die positiven wie auch die negativen Ionen sind überwiegend einwertig, möglich sind jedoch auch multivalente Anionen und/oder Kationen, beispielsweise mit einer bis fünf, vorzugsweise mit einer bis vier, besonders bevorzugt mit einer bis drei, insbesondere mit einer bis zwei, elektrischen Ladungen pro Ion. Die Ladungen können sich an verschiedenen lokalisierten oder delokalisierten Bereichen innerhalb eines Moleküls befinden, also betainartig vorliegen, oder auch wie ein getrenntes Anion und Kation verteilt sein. Bevorzugt sind solche ionischen Flüssigkeiten, die aus mindestens einem Kation und mindestens einem Anion aufgebaut sind.

Die vorliegende Erfindung ist nicht auf spezielle ionische Flüssigkeiten beschränkt; es können alle geeigneten ionischen Flüssigkeiten verwendet werden, worunter auch Gemische verschiedener ionischer Flüssigkeiten verstanden werden, beispielsweise auch Mischungen aus herkömmlichen festen, gasförmigen oder flüssigen Schleppmitteln zur Anreicherung einer Komponente eines Gemisches, wie n-Methylpyrrolidon, Dimethylformamid, Ethandiol, Benzol, Cyclohexan, Wasser etc., mit ionischen Flüssigkeiten.

Bevorzugt sind ionische Flüssigkeiten mit möglichst niedrigem Schmelzpunkt, besonders bevorzugt unterhalb von 200°C, insbesondere unterhalb von 100°C, speziell unterhalb von 76°C.

Die ionischen Flüssigkeiten weisen eine Molmasse von vorzugsweise höchstens 1.000 g/mol, besonders bevorzugt höchstens 500 g/mol, auf.

Bevorzugt als Kation sind Phosphoniumionen oder solche Kationen, die mindestens einen fünf- oder sechsgliedrigen Heterocyclus enthalten, der mindestens ein Phosphor- oder Stickstoffatom sowie gegebenenfalls ein Sauerstoff- oder Schwefelatom aufweist. Besonders bevorzugt sind Kationen, die mindestens einen fünf- oder sechsgliedrigen Heterocyclus enthalten, der ein, zwei oder drei Stickstoffatome und ein Schwefel- oder ein Sauerstoffatom aufweist. Ganz besonders bevorzugt sind Kationen, die mindestens einen fünf- oder sechsgliedrigen Heterocyclus enthalten, der ein oder zwei Stickstoffatome aufweist.

Weiterhin sind solche Kationen bevorzugt, die ausgewählt sind aus den Verbindungen der Formeln (Ia) bis (Iw), 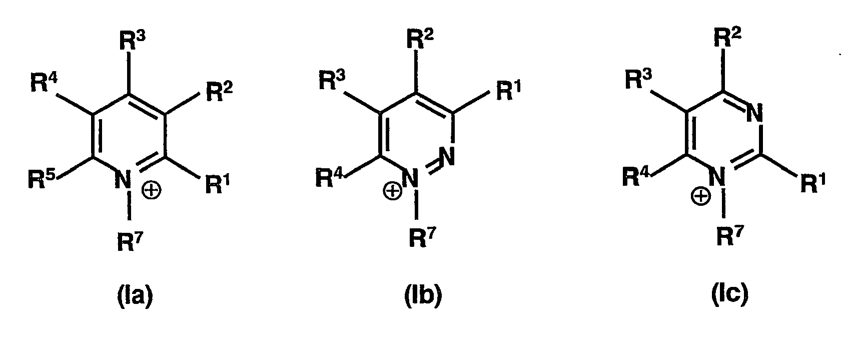 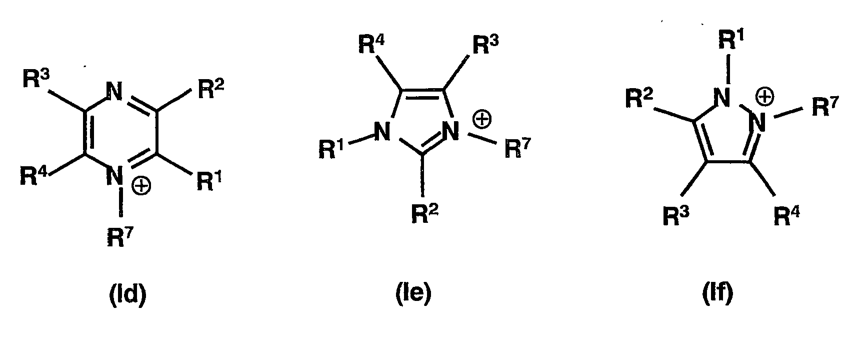 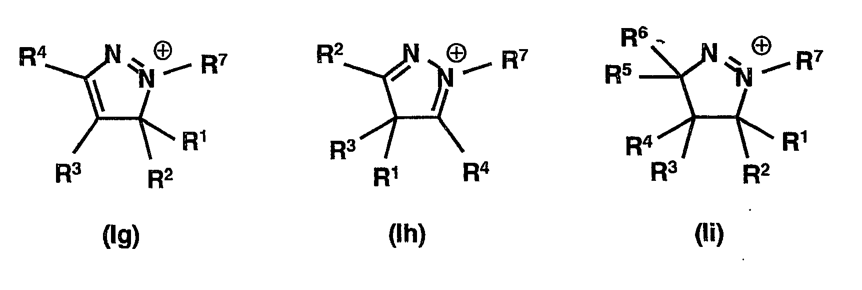 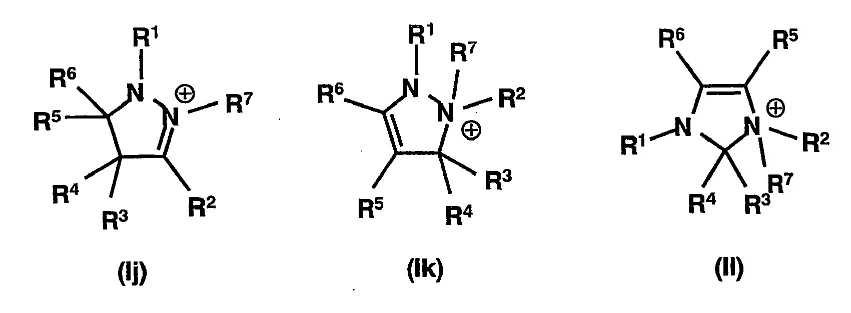 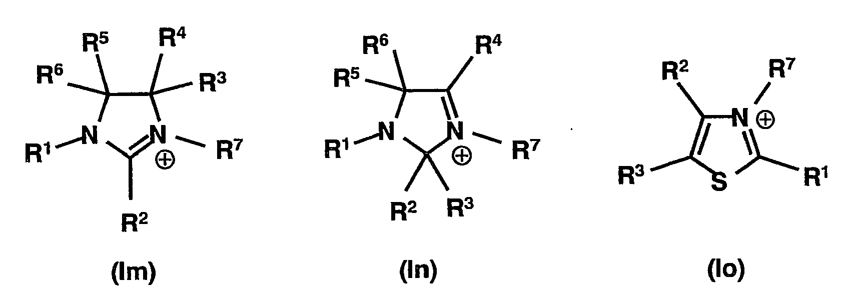 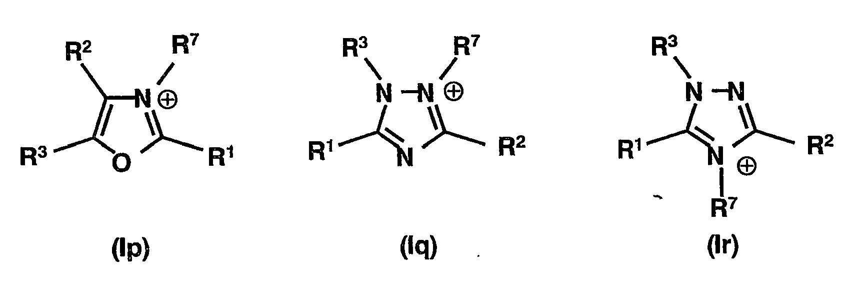 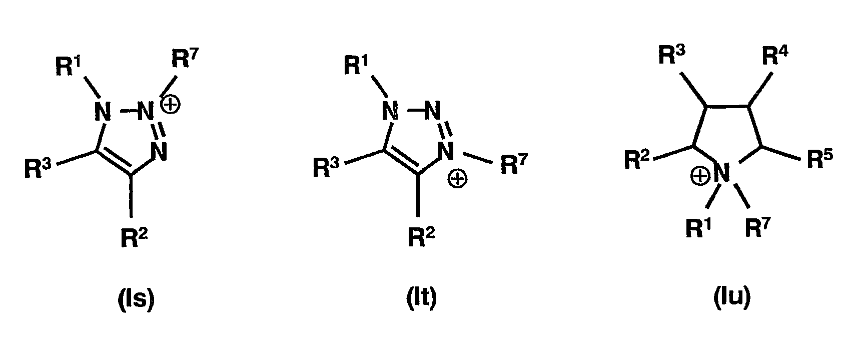 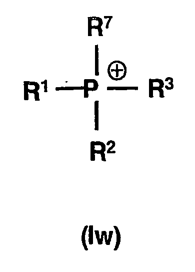 sowie Oligo- bzw. Polymere, die diese Strukturen enthalten,
worin
die Reste R¹, R², R³, R⁴, R⁵, R⁶ und R⁷, jeweils unabhängig voneinander Wasserstoff, gegebenenfalls durch funktionelle Gruppen, Aryl, Alkyl, Aryloxy, Alkyloxy, Halogen, Heteroatome und/oder Heterocyclen substituiertes C₁-C₁₈-Alkyl, gegebenenfalls durch ein oder mehrere nicht benachbarte Sauerstoff- und/oder Schwefelatome und/ oder ein oder mehrere substituierte oder unsubstituierte Iminogruppen unterbrochenes C₂-C₁₈-Alkyl, C₆-C₁₂-Aryl, C₅-C₁₂-Cycloalkyl oder einen fünf- bis sechsgliedrigen, Sauerstoff , Stickstoff- und/oder Schwefelatome aufweisenden Heterocyclus bedeuten oder zwei von den Resten gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die Reste jeweils durch funktionelle Gruppen, Aryl, Alkyl, Aryloxy, Alkyloxy, Halogen, Heteroatome und/oder Heterocyclen substituiert sein können.

R⁷ kann darüber hinaus C₁-C₁₈-Alkyloyl (Alkylcarbonyl), C₁-C₁₈- Alkyloxycarbonyl, C₅-C₁₂-Cycloalkylcarbonyl oder C₆-C₁₂-Aryloyl (Arylcarbonyl) bedeuten, wobei die genannten Reste jeweils durch funktionelle Gruppen, Aryl, Alkyl, Aryloxy, Alkyloxy, Halogen, Heteroatome und/oder Heterocyclen substituiert sein können, wobei sich die Angaben C₁-C₁₈ auf Alkyl bezieht.

Darin bedeuten
gegebenenfalls durch funktionelle Gruppen, Aryl, Alkyl, Aryloxy, Alkyloxy, Halogen, Heteroatome und/oder Heterocyclen substituiertes C₁-C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hetadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Buty phenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl und, gegebenenfalls durch ein oder mehrere nicht benachbarte Sauerstoff- und/oder Schwefelatome und/oder ein oder mehrere substituierte oder unsubstituierte Iminogruppen unterbrochenes C₂-C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bilden zwei Reste einen Ring, so können diese Reste gemeinsam bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Die Anzahl der Sauerstoff- und/oder Schwefelatome und/oder Iminogruppen in der ionischen Flüssigkeit ist nicht beschränkt. Im Allgemeinen beträgt sie nicht mehr als 5 in dem Rest, vorzugsweise nicht mehr als 4, insbesondere nicht mehr als 3.

Weiterhin befindet sich zwischen zwei Heteroatomen mindestens ein Kohlenstoffatom, besonders bevorzugt mindestens zwei.

Substituierte und unsubstituierte Iminogruppen können beispielsweise Imino-, Methylimino-, iso-Propylimino, n-Butylimino oder tert-Butylimino sein.

Weiterhin bedeuten
funktionelle Gruppen Carboxy, Carboxamid, Hydroxy, Di-(C₁-C₄-Alkyl)-amino, C₁-C₄-Alkyloxycarbonyl, Cyano oder C₁-C₄-Alkyloxy,
gegebenenfalls durch funktionelle Gruppen, Aryl, Alkyl, Aryloxy, Alkyloxy, Halogen, Heteroatome und/oder Heterocyclen substituiertes C₆-C₁₂-Aryl beispielsweise Phenyl, Tolyl, Xylyl, α-Naphthyl, β-naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, I-sopropylnaphthyl, Chlornaphthyl, Ethoxynaphthyl, 2,6-Dimethylphenyl, 2,4,6-Trimethylphenyl, 2,6-Diethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxyethylphenyl,
gegebenenfalls durch funktionelle Gruppen, Aryl, Alkyl, Aryloxy, Alkyloxy, Halogen, Heteroatome und/oder Heterocyclen substituiertes C₅-C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl, ein fünf- bis sechsgliedriger, Sauerstoff-, Stickstoff- und/oder Schwefelatome aufweisender Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl und

C₁ bis C₄-Alkyl beispielsweise Methyl, Ethyl, Propyl, Isopropyl, n-Butyl, sec-Butyl oder tert.-Butyl.

C₁-C₁₈-Alkyloyl (Alkylcarbonyl) kann beispielsweise sein Acetyl, Propionyl, n-Butyloyl, sec-Butyloyl, tert.-Butyloyl, 2-Etylhexylcarbonyl, Decanoyl, Dodecanoyl, Chloracetyl, Trichloracetyl oder Trifluoracetyl.

C₁-C₁₈- Alkyloxycarbonyl kann beispielsweise sein Methyloxycarbonyl, Ethyloxycarbonyl, Propyloxycarbonyl, Isopropyloxycarbonyl, n-Butyloxycarbonyl, sec-Butyloxycarbonyl, tert.-Butyloxycarbonyl, Hexyloxycarbonyl, 2-Etylhexyloxycarbonyl oder Benzyloxycarbonyl.

C₅-C₁₂-Cycloalkylcarbonyl kann beispielsweise sein Cyclopentylcarbonyl, Cyclohexylcarbonyl oder Cyclododecylcarbonyl.

C₆-C₁₂-Aryloyl (Arylcarbonyl) kann beispielsweise sein Benzoyl, Toluyl, Xyloyl, α-Naphthoyl, β-Naphthoyl, Chlorbenzoyl, Dichlorbenzoyl, Trichlorbenzoyl oder Trimethylbenzoyl.

Bevorzugt sind R¹, R², R³, R⁴, R⁵ und R⁶, jeweils unabhängig voneinander, Wasserstoff, Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino und Chlor.

Bevorzugt ist R⁷ Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Acetyl, Propionyl, t-Butyryl, Methoxycarbonyl, Ethoxycarbonyl oder n-Butoxycarbonyl.

Besonders bevorzugte Pyridiniumionen (Ia) sind solche, bei denen einer der Reste R¹ bis R⁵ Methyl, Ethyl oder Chlor ist, R⁷ Acetyl, Methyl, Ethyl oder n-Butyl und alle anderen Reste Wasserstoff sind, oder R³ Dimethylamino, R⁷ Acetyl, Methyl, Ethyl oder n-Butyl und alle anderen Reste Wasserstoff sind oder R⁷ Acetyl, Methyl, Ethyl oder n-Butyl und alle anderen Reste Wasserstoff sind oder R² Carboxy oder Carboxamid, R⁷ Acetyl, Methyl, Ethyl oder n-Butyl und alle anderen Reste Wasserstoff sind oder R¹ und R² oder R² und R³ 1,4-Buta-1,3-dienylen, R⁷ Acetyl, Methyl, Ethyl oder n-Butyl und alle anderen Reste Wasserstoff sind.

Besonders bevorzugte Pyridaziniumionen (Ib) sind solche, bei denen einer der Reste R¹ bis R⁴ Methyl oder Ethyl, R⁷ Acetyl, Methyl, Ethyl oder n-Butyl und alle anderen Reste Wasserstoff oder R⁷ Acetyl, Methyl, Ethyl oder n-Butyl, und alle anderen Reste Wasserstoff sind.

Besonders bevorzugte Pyrimidiniumionen (Ic) sind solche, bei denen R² bis R⁴ Wasserstoff oder Methyl, R⁷ Acetyl, Methyl, Ethyl oder n-Butyl und R¹ Wasserstoff, Methyl oder Ethyl ist, oder R² und R⁴ Methyl, R³ Wasserstoff und R¹ Wasserstoff, Methyl oder Ethyl und R⁷ Acetyl, Methyl, Ethyl oder n-Butyl ist.

Besonders bevorzugte Pyraziniumionen (Id) sind solche, bei denen
R¹ bis R⁴ alle Methyl und
R⁷ Acetyl, Methyl, Ethyl oder n-Butyl oder R⁷ Acetyl, Methyl, Ethyl oder n-Butyl und alle anderen Reste Wasserstoff sind.

Besonders bevorzugte Imidazoliumionen (Ie) sind solche, bei denen unabhängig voneinander
R¹ ausgewählt ist aus der Gruppe bestehend aus Methyl, Ethyl, n-Propyl, n-Butyl, n-Pentyl, n-Octyl, n-Decyl, n-Dodecyl, 2-Hydroxyethyl und 2-Cyanoethyl,
R⁷ Acetyl, Methyl, Ethyl oder n-Butyl und
R² bis R⁴ unabhängig voneinander Wasserstoff, Methyl oder Ethyl bedeuten.

Besonders bevorzugte 1H-Pyrazoliumionen (If) sind solche, bei denen unabhängig voneinander
R¹ unter Wasserstoff, Methyl oder Ethyl,
R², R³ und R⁴ unter Wasserstoff oder Methyl und
R⁷ unter Acetyl, Methyl, Ethyl oder n-Butyl ausgewählt sind.
Besonders bevorzugte 3H-Pyrazoliumionen (Ig) sind solche, bei denen unabhängig voneinander
R¹ unter Wasserstoff, Methyl oder Ethyl,
R², R³ und R⁴ unter Wasserstoff oder Methyl und
R⁷ unter Acetyl, Methyl, Ethyl oder n-Butyl ausgewählt sind.

Besonders bevorzugte 4H-Pyrazoliumionen (Ih) sind solche, bei denen unabhängig voneinander
R¹ bis R⁴ unter Wasserstoff oder Methyl und
R⁷ unter Acetyl, Methyl, Ethyl oder n-Butyl ausgewählt sind.
Besonders bevorzugte 1-Pyrazoliniumionen (Ii) sind solche, bei denen unabhängig voneinander

R¹ bis R⁶ unter Wasserstoff oder Methyl und
R⁷ unter Acetyl, Methyl, Ethyl oder n-Butyl ausgewählt sind.

Besonders bevorzugte 2-Pyrazoliniumionen (Ij) sind solche, bei denen unabhängig voneinander
R¹ unter Wasserstoff, Methyl, Ethyl oder Phenyl,
R⁷ unter Acetyl, Methyl, Ethyl oder n-Butyl und
R² bis R⁶ unter Wasserstoff oder Methyl ausgewählt sind.

Besonders bevorzugte 3-Pyrazoliniumionen (Ik) sind solche, bei denen unabhängig voneinander
R¹ oder R² unter Wasserstoff, Methyl, Ethyl oder Phenyl,
R⁷ unter Acetyl, Methyl, Ethyl oder n-Butyl und
R³ bis R⁶ unter Wasserstoff oder Methyl ausgewählt sind.

Besonders bevorzugte Imidazoliniumionen (II) sind solche, bei denen unabhängig voneinander
R¹ oder R² unter Wasserstoff, Methyl, Ethyl, n-Butyl oder Phenyl,
R⁷ unter Acetyl, Methyl, Ethyl oder n-Butyl und
R³ oder R⁴ unter Wasserstoff, Methyl oder Ethyl und
R⁵ oder R⁶ unter Wasserstoff oder Methyl ausgewählt sind.

Besonders bevorzugte Imidazoliniumionen (Im) sind solche, bei denen unabhängig voneinander
R¹ oder R² unter Wasserstoff, Methyl oder Ethyl,
R⁷ unter Acetyl, Methyl, Ethyl oder n-Butyl und
R³ bis R⁶ unter Wasserstoff oder Methyl ausgewählt sind.
Besonders bevorzugte Imidazoliniumionen (In) sind solche, bei denen unabhängig voneinander
R¹, R² oder R³ unter Wasserstoff, Methyl oder Ethyl,
R⁷ Acetyl, Methyl, Ethyl oder n-Butyl und
R⁴ bis R⁶ unter Wasserstoff oder Methyl ausgewählt sind.

Besonders bevorzugte Thiazoliumionen (Io) oder Oxazoliumionen (Ip) sind solche, bei denen unabhängig voneinander
R¹ unter Wasserstoff, Methyl, Ethyl oder Phenyl,
R⁷ unter Acetyl, Methyl, Ethyl oder n-Butyl und
R² oder R³ unter Wasserstoff oder Methyl ausgewählt sind.

Besonders bevorzugte 1,2,4-Triazoliumionen (Iq) und (Ir) sind solche, bei denen unabhängig voneinander
R¹ oder R² unter Wasserstoff, Methyl, Ethyl oder Phenyl,
R⁷ unter Acetyl, Methyl, Ethyl oder n-Butyl und
R³ unter Wasserstoff, Methyl oder Phenyl ausgewählt sind.

Besonders bevorzugte 1,2,3-Triazoliumionen (Is) und (It) sind solche, bei denen unabhängig voneinander
R¹ unter Wasserstoff, Methyl oder Ethyl,
R⁷ unter Acetyl, Methyl, Ethyl oder n-Butyl und
R² oder R³ unter Wasserstoff oder Methyl ausgewählt sind oder
R² und R³ 1,4-Buta-1,3-dienylen und alle anderen Wasserstoff sind.

Besonders bevorzugte Pyrrolidiniumionen (Iu) sind solche, bei denen unabhängig voneinander
R¹ und R⁷ unter Acetyl, Methyl, Ethyl oder n-Butyl ausgewählt sind und
R², R³, R⁴ und R⁵ Wasserstoff bedeuten.

Besonders bevorzugte Phosphoniumionen (Iw) sind solche, bei denen unabhängig voneinander
R⁷ unter Acetyl, Methyl, Ethyl oder n-Butyl und
R¹, R², und R³ unter Phenyl, Phenoxy, Ethoxy und n-Butoxy ausgewählt sind.
Unter den zuvor erwähnten Ionen sind, Phosphonium-, Pyridinium- und Imidazoliumionen bevorzugt.

Ganz besonders bevorzugt sind als Kationen 1,2-Dimethylpyridinium, 1-Methyl-2-ethylpyridinium, 1-Methyl-2-ethyl-6-methylpyridinium, N-Methylpyridinium, 1-Butyl-2-methylpyridinium, 1-Butyl-2-ethylpyridinium, 1-Butyl-2-ethyl-6-methylpyridinium, N-Butylpyridinium, 1-Butyl-4-methylpyridinium, 1,3-Dimethylimidazolium, 1,2,3-Trimethylimidazolium, 1-n-Butyl-3-methylimidazolium, 1,3,4,5-Tetramethylimidazolium, 1,3,4-Trimethylimidazolium, 2,3-Dimethylimidazolium, 1-Butyl-2,3-dimethylimidazolium, 3,4-Dimethylimidazolium, 2-Ethyl-3,4-dimethylimidazolium, 3-Methyl-2-ethylimidazol, 3-Butyl-1-methylimidazolium, 3-Butyl-1-ethylimidazolium, 3-Butyl-1,2-dimethylimidazolium, 1,3-Di-n-Butylimidazolium, 3-Butyl-1,4,5-Trimethylimidazolium, 3-Butyl-1,4-Dimethylimidazolium, 3-Butyl-2-methylimidazolium, 1,3-Dibutyl-2-methylimidazolium, 3-Butyl-4-methylimidazolium, 3-Butyl-2-ethyl-4-methylimidazolium und 3-Butyl-2-ethylimidazolium, 1-Methyl-3-Octylimidazolium, 1-Decyl-3-Methylimidazolium.

Insbesondere bevorzugt sind 1-Butyl-4-methylpyridinium, 1-n-Butyl-3-methylimidazolium und 1-n-Butyl-3-ethylimidazolium.

Auch möglich sind Kationen, die von Diazabicyclononen oder Diazabicycloundecen abgeleitet werden, sowie deren Mischungen oder Derivate.

Als Anionen sind prinzipiell alle Anionen denkbar.

Bevorzugt als Anionen sind Halogenide F⁻, Cl⁻, Br⁻, I⁻, Acetat CH₃COOO⁻, Trifluoracetat CF₃COO⁻, Triflat CF₃SO₃⁻, Sulfat SO₄²⁻, Hydrogensulfat HSO₄⁻, Methylsulfat CH₃OSO₃⁻, Ethylsulfat C₂H₅OSO₃⁻, Sulfit SO₃²⁻, Hydrogensulfat HSO₃⁻, Aluminiumchloride AlCl₄⁻, Al₂Cl₇⁻, Al₃Cl₁₀⁻, Aluminiumtribromid AlBr₄⁻, Nitrit NO₂⁻, Nitrat NO₃⁻, Kupferchlorid CuCl₂⁻ , Phosphate, Phosphat PO₄³⁻, Hydrogenphosphat HPO₄²-, Dihydrogenphosphat H₂PO₄⁻, Carbonat CO₃²⁻, Hydrogencarbonat HCO₃⁻, Sulfonat -SO₃⁻, Tosylat p-CH₃C₆H₄SO₃⁻ bzw. Bis(trifluormethylsulfonyl)imid (CF₃SO₂)₂N⁻.

### Heterogener Katalysator

Der in dem erfindungsgemäßen Verfahren verwendete heterogene Katalysator kann ein Trägermaterial enthalten oder als Vollkontakt verwendet werden. Darüber hinaus kann der heterogene Katalysator als Pulver (Suspensionsfahrweise) oder als Formkörper (Festbett) vorliegen. Typische Formkörper sind Kugeln, Stränge, Hohlstränge, Sternstränge, Tabletten, Splitt, etc. mit charakteristischen Durchmessern von 0,5 bis 5 mm oder auch Monolithe und ähnliche strukturierte Packungen (vgl. Ullmann's Encyclopedia, Sixth Edition, 2000 Electronic Release, Chapter Fixed-Bed Reactors, Par. 2: Catalyst Forms for Fixed-Bed Reactors).
Die vorliegende Erfindung ist nicht eingeschränkt auf spezielle heterogene Katalysatoren; es können alle geeigneten heterogenen Katalysatoren verwendet werden. Geeignete Katalysatoren enthalten beispielsweise Metalle, die ausgewählt sind aus der Gruppe, bestehend aus Nickel, Kobalt, Kupfer, Eisen, Ruthenium, Rhodium, Iridium, Palladium und Platin. Die zuvor genannten Metall-Katalysatoren können gegebenenfalls auch als Skelett-Katalysatoren ausgeführt sein . Die heterogenen Katalysatoren können dotiert oder undotiert sein. Geeignete Dotiermetalle können ausgewählt werden aus den Elementen der Gruppen 3 bis 12 des Periodensystems der Elemente gemäß IUPAC-Nomenklatur (Handbook of Chemistry and Physics, 80. Ausgabe, 1999-2000).

Darüber hinaus können auch Kombinationen der zuvor erwähnten Materialien als heterogener Katalysator verwendet werden.

Wenn in dem erfindungsgemäßen Verfahren ein geträgerter heterogener Katalysator verwendet wird, so ist man im vorliegenden Verfahren nicht auf spezielle Trägermaterialien beschränkt. Beispielsweise können Ruße, Acetylenschwarz, Kohle, Graphit, SiO₂, Al₂O₃, ZrO₂, ZnO₂, TiO₂, MgO, Zeolithe, Hydrotalcite oder weitere, dem Fachmann an sich bekannte Trägermaterialien in den verschiedenen möglichen Modifikationen verwendet werden. Die Trägermaterialien können zusätzlich, etwa mit Alkali- oder Erdalkalimetallen oder auch mit Phosphor-, Halogenid- und/oder Sulfatsalzen dotiert sein. Im Allgemeinen werden die Säure-Baseeigenschaften durch solche Dotierungen modifiziert, was sich positiv auf die katalytischen Eigenschaften auswirken kann. Die zuvor genannten hydrieraktiven Metalle können durch jedes geeignete Verfahren auf dem Träger aufgebracht werden, beispielsweise durch Imprägnieren, Ionenaustausch, Coprezipitation, z.B. gemeinsame Fällung mit dem Träger, Fällung auf einen vorgelegten Träger, Ionenaustausch, Chemical Vapour Deposition (CVD) etc.

Wenn in dem erfindungsgemäßen Verfahren ein geträgerter heterogener Katalysator verwendet wird, so ist das katalytisch wirkende Metall in einer Menge von vorzugsweise 0,1 bis 60 Gew.-%, besonders bevorzugt 1 bis 50 Gew.-%, insbesonders 2 bis 50 Gew.-%, jeweils bezogen auf den gesamten Katalysator, vorhanden.

Wird der heterogene Katalysator als Formkörper, etwa für ein Festbettverfahren, hergestellt, kann er in jeder beliebigen Form verwendet werden. Typische Formkörper sind Kugeln, Stränge, Hohlstränge, Sternstränge, Tabletten, Splitt, etc. mit charakteristischen Durchmessern von 0,5 bis 5 mm, oder auch Monolithe und ähnliche strukturierte Packungen (vgl. Ullmann's Encyclopedia, Sixth Edition, 2000 Electronic Release, Chapter Fixed-Bed Reactors, Par. 2: Catalyst Forms for Fixed-Bed Reactors). Bei der Suspensionsfahrweise wird der Katalysator in Pulverform eingesetzt. Typische Partikelgrößen in solchen Pulvern liegen bei 1-100µm, es können aber auch Partikel deutlich kleiner als 1µm verwendet werden, etwa beim Einsatz von Ruß als Katalysatorträger. Die Filtration kann in Suspensionsverfahren diskontinuierlich, etwa durch Tiefenfiltration durchgeführt werden. In kontinuierlichen Verfahren bietet sich etwa die Querstromfiltration an.

Das molare Verhältnis von Katalysator zu Nitril bzw. Dinitril kann im Allgemeinen beliebig sein, solange eine Hydrierung des Nitrils bzw. Dinitrils erfolgt. Das Gewichtsverhältnis von Katalysator zu Nitril bzw. Dinitril ist vorzugsweise 0.0001:1 bis 1:1, besonders bevorzugt 0.001:1 bis 0,25:1.

In dem erfindungsgemäßen Verfahren der Hydrierung von Nitrilen wird der heterogene Katalysator in Kombination mit einer ionischen Flüssigkeit verwendet. Dabei ist es bevorzugt, dass die Polarität des heterogenen Katalysators und die Polarität der ionischen Flüssigkeit aufeinander abgestimmt sind. Zur Definition von Polaritäten ionischer Flüssigkeiten wird dabei auf: P. Wasserscheid, T. Welton (Hrsg.), Ionic Liquids in Synthesis, Wiley VCH, Weinheim 2003, Seite 94 ff. verwiesen.

So ist bei einem heterogenen Katalysator mit polarer Oberfläche eine unpolare ionische Flüssigkeit und bei einem heterogenen Katalysator mit einer unpolaren Oberfläche eine polare ionische Flüssigkeit bevorzugt. Bevorzugt ist auch, dass die ionische Flüssigkeit und Katalysator so gewählt werden, dass sich Edukt oder Produkt in einer anderen Phase aufhalten. Bevorzugt ist ferner, dass durch die ionische Flüssigkeit eine irreversible Belegung des Katalysators verhindert wird. -

Demnach werden in einer ersten Ausführungsform der vorliegenden Erfindung ein heterogener Katalysator mit einer unpolaren Oberfläche und eine polare ionische Flüssigkeit verwendet. In dieser Ausführungsform wird der Katalysator von der ionischen Flüssigkeit im Wesentlichen nicht benetzt.

In einer zweiten Ausführungsform des erfindungsgemäßen Verfahrens ist die Oberfläche des heterogenen Katalysators polar und wird nicht von der vergleichsweise unpolaren ionischen Flüssigkeit benetzt.

Das erfindungsgemäße Verfahren weist durch die zuvor beschriebene Wahl geeigneter ionischer Flüssigkeiten und geeigneter heterogener Katalysatoren eine hohe Selektivität auf, die beispielsweise dadurch erreicht wird, dass eine der beteiligten Komponenten, beispielsweise das hydrierte Amin aus den reaktiven Bedingungen der Hydrierung hinein in eine andere Phase (auch zum Beispiel in Form von kleinsten Tröpfchen in einer Emulsion vorliegend) entfernt wird. Damit wird eine weitere katalytische Umsetzung des hergestellten Produkts unterbunden. Die Selektivität wird somit durch die räumliche Trennung der beteiligten Komponenten erhöht.

In einer dritten Ausführungsform des erfindungsgemäßen Verfahrens kann die Oberfläche des Katalysators polar sein und von der polaren ionischen Flüssigkeit benetzt werden, so dass ein unpolareres Edukt bzw. Produkt eine zweite Phase bildet und so vom aktiven Katalysator getrennt werden kann, was die Bildung von Nebenprodukten gegebenenfalls erniedrigt.

In einer vierten Ausführungsform des erfindungsgemäßen Verfahrens können ein heterogener Katalysator mit unpolarer Oberfläche und eine vergleichsweise unpolare ionische Flüssigkeit verwendet werden. In diesem Fall können polareres Edukt bzw. Produkt eine zweite Phase bilden und so vom aktiven Katalysator getrennt werden.

In einer fünften Ausführungsform des erfindungsgemäßen Verfahrens kann eine irreversible Belegung der Oberfläche des Katalysators durch Nebenkomponenten durch eine reversibel koordinierend wirkende ionische Flüssigkeit verhindert werden.

In einer sechsten Ausführungsform des erfindungsgemäßen Verfahrens kann eine selbst nur schwach koordinierende ionische Flüssigkeit durch die Erzeugung einer polaren, ionischen Umgebung eine Belegung der Katalysatoroberfläche lurch Nebenkomponenten verhindern (Spüleffekt).

Im Idealfall koordiniert beispielsweise die ionische Flüssigkeit stärker als das Aminendprodukt an der Katalysatoroberfläche und verdrängt dieses somit, wodurch Nebenreaktionen verhindert werden. Die Koordination ist jedoch ähnlich bzw. schwächer als die des Eduktes, so dass ein Austausch der ionischen Flüssigkeit durch das Edukt möglich ist, das daraufhin an der Katalysatoroberfläche umgesetzt wird.

Sofern die ionische Flüssigkeit Koordinationseffekte ausübt, ist es vorteilhaft, aber keine zwingende Voraussetzung, dass eine ausreichende Zugänglichkeit der Katalysatoroberfläche für die Moleküle der ionischen Flüssigkeit gewährleistet wird.

### Hydrierung

Zur Durchführung des erfindungsgemäßen Verfahrens ist es vorteilhaft, wenn neben der Polarität die Löslichkeit der verschiedenen Komponenten in den beteiligten Lösemitteln bzw. ionischen Flüssigkeiten, die Viskositäten und Dichten und die Gasflüssigkeiten bzw. Diffusionsgeschwindigkeiten der beteiligten Gase in den verschiedenen Phasen berücksichtigt werden.

Die Hydrierung des erfindungsgemäßen Verfahrens kann mit Rückführung oder ohne Rückführung (gerader Durchgang) erfolgen. Die Hydrierung kann darüber hinaus in kontinuierlicher oder diskontinuierlicher Betriebsweise durchgeführt werden. Dabei kann Vollumsatz oder zum Beispiel durch vorzeitiges Abbrechen Teilumsatz erzeugt werden. Eine Rückvermischung ist in dem erfindungsgemäßen Verfahren möglich, aber nicht zwingend erforderlich. Das erfindungsgemäße Verfahren kann in jeder geeigneten, dem Fachmann bekannten Vorrichtung durchgeführt werden. Geeignet sind beispielsweise Rohrreaktoren, Blasensäulen, Autoklaven, druckfeste Rührbehälter oder Reaktorkaskaden. Die Hydrierung kann dabei in einer einzigen Vorrichtung oder in mehreren hintereinander geschalteten Vorrichtungen, beispielsweise in Rieselbett- oder Sumpffahrweise durchgeführt werden.

Der Druck, bei dem das erfindungsgemäße Verfahren durchgeführt wird, beträgt vorzugsweise 1 bis 300 bar, besonders bevorzugt 1 bis 200, insbesondere 1 bis 150. Die Hydrierung wird vorzugsweise bei einer Temperatur von mindestens 20°C, insbesondere 50°C durchgeführt. Die Hydrierung wird vorzugsweise bei einer Temperatur von höchstens 250°C, besonders bevorzugt höchstens 200°C, insbesondere höchstens 150°C, durchgeführt.

Das erfindungsgemäße Verfahren eignet sich in einer besonders bevorzugten Ausführungsform zur Hydrierung von Nitrilfunktionen in einer solchen organischen Verbindung, die mindestens zwei Nitrilfunktionen aufweist. Diesbezüglich ist insbesondere die Herstellung von Aminonitrilen ausgehend von Dinitrilen zu erwähnen. Beispiel hierfür ist die Hydrierung von Adipodinitril zu Aminocapronitril, bei der Hexamethylendiamin nur im Unterschuss gebildet wird und der Anteil des Diamins sich je nach Wahl der Reaktionspartner und Reaktionsbedingungen steuern läßt Ebenso eignet es sich in einer weiteren besonders bevorzugten Ausführungsform zur Hydrierung von Nitrilfunktionen in labilen organischen Verbindungen, die sich unter normalen Reaktionsbedingungen, z.B. in Gegenwart des Hydrierproduktes zersetzten.

Beispiel hierfür ist die Hydrierung von Iminodiacetonitril zu Diethylentriamin, bei der Diethylentriamin mit hoher Selektivität erzeugt werden kann.

Es ist möglich, das erfindungsgemäße Verfahren in Gegenwart von Ammoniak durchzuführen, beispielsweise bei einem Stoffmengenverhältnis von Ammoniak zu Nitril von 10 bis 1, insbesondere 2 bis 1. In einer besonders bevorzugten Ausführungsform des erfindungsgemäßen Verfahrens findet die Hydrierung jedoch in Abwesenheit von Ammoniak statt.

Zur Hydrierung kann jedes Wasserstoff enthaltende Fluid verwendet werden, soweit der Gehalt an Wasserstoff bzw. die Nachdiffusion des Wasserstoffs in dem Fluid ausreichend ist, um Nitrilfunktionen zu hydrieren. Die Reaktionszeit der Hydrierung ist abhängig von dem zu hydrierenden Substrat, dem verwendeten Katalysator und den Hydrierbedingungen. Sie kann beispielsweise einige Minuten bis einige Stunden betragen. Das zur Hydrierung verwendete Gas enthält vorzugsweise 1 bis 100 Vol.%, besonders bevorzugt 50 bis 100 Vol.%, insbesondere 90 bis 100 Vol.%, Wasserstoff. In einer besonders bevorzugten Ausführungsform wird reiner Wasserstoff verwendet.

Das molare Verhältnis von Wasserstoff, gegebenenfalls in dem Fluid, zum Nitril bzw. Dinitril ist nicht kritisch, solange ausreichend Wasserstoff vorhanden ist, die Nitrilfunktion zu hydrieren. Im Allgemeinen wird Wasserstoff im Überschuss verwendet.

Die Aufarbeitung des aus dem erfindungsgemäßen Verfahren stammenden Reaktionsaustrags, d.h. des Katalysators (im Falle einer Suspensionsfahrweise), der ionischen Flüssigkeit und der Edukte bzw. Produkte, kann je nach Prozessanforderungen für jede einzelne Komponente getrennt oder zusammen erfolgen, z.B. durch eine besonders bevorzugte und einfache Destillation der Edukte, Wertprodukte und Verunreinigungen bzw. Nebenprodukte. Eine weitere bevorzugte Aufarbeitungsmöglichkeit besteht in der Extraktion der Reaktionspartner zur Trennung von der ionischen Flüssigkeit. Zur Abtrennung des Katalysators von der ionischen Flüssigkeit und dem Reaktionsgemisch ist gegebenenfalls auch eine Filtration möglich. Edukte und/oder Produkte können von der ionischen Flüssigkeit und/oder dem Katalysator auch durch einfache Phasentrennung separiert werden. Falls ein konventionelles Lösemittel zusätzlich zu der ionischen Flüssigkeit verwendet wird, so kann dessen Trennung von der ionischen Flüssigkeit beispielsweise durch Destillation erfolgen. Falls die ionische Flüssigkeit und das konventionelle Lösemittel nicht miteinander mischbar sind, ist auch eine einfache Phasentrennung der ionischen Flüssigkeit von dem konventionellen Lösemittel möglich.

Im Falle einer Suspensionsfahrweise können Katalysator und ionische Flüssigkeit getrennt oder zusammen zurückgeführt werden. In einer Festbettfahrweise kann die ionische Flüssigkeit in das Verfahren zurückgeführt werden.

Die ionische Flüssigkeit ist vorzugsweise im Kreis zu führen. Um Fremdstoffe, die sich in der ionischen Flüssigkeit anreichern, zu entfernen, kann dabei ein Teil der ionischen Flüssigkeit vorzugsweise als Purge-Strom aus dem System ausgeschleust und durch eine frische ionische Flüssigkeit ersetzt werden. Die Menge an Purge-Strom beträgt vorzugsweise 0 bis 15 Gew.-%, besonders bevorzugt weniger als 10 Gew.-%, insbesondere weniger 5 Gew.-%.

Weitere Varianten zur Aufarbeitung der ionischen Flüssigkeit an Stelle des Ausschleusens sind beispielsweise:
- Flüssig-flüssig-Extraktion mit nicht mischbaren Lösemitteln, beispielsweise Wasser, organischen Lösemitteln und Säuren (in Abhängigkeit davon, ob die ionische Flüssigkeit wasserlöslich oder -unlöslich ist),
- Umkristallisation,
- Membranpermeation bzw. Filtration und
- Strippen mit einem inerten Gas beispielsweise Stickstoff.
- Reaktion unter Spaltung der ionischen Flüssigkeit, Destillation oder Extraktion der Komponenten und Reformierung der ionischen Flüssigkeit.

Im Idealfall ist eine Aufarbeitung der ionischen Flüssigkeit nicht nötig, da die Reaktionskomponenten bzw. die Reaktionsbedingungen so aufeinander abgestimmt werden, daß keine Anreicherung von Reaktionskomponenten bzw. Nebenprodukten auftritt, die eine Aufarbeitung nötig macht.

Im erfindungsgemäßen Verfahren werden Nitrilfunktionen, die in organischen Verbindungen enthalten sind, hydriert. Die organischen Verbindungen enthalten dabei vorzugsweise eine, zwei oder drei Nitrilfunktionen.

Besonders bevorzugt werden organische Verbindungen mit den folgenden Struktureinheiten hydriert 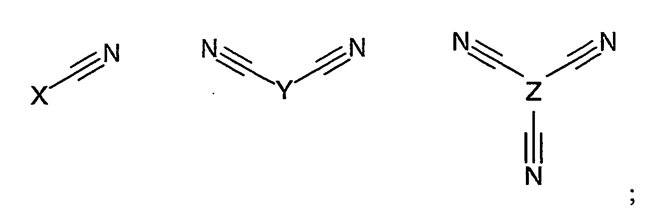 wobei in den Struktureinheiten
X für lineare, verzweigte oder cyclische Gruppen steht, die ausgewählt sind aus der Gruppe, bestehend aus Alkyl, Cycloalkyl, Alkenyl, Alkinyl, Aryl, Hydroxyalkyl, Alkoxyalkyl, Aminoalkyl und Aryl-C₁₋₄,
Y und Z ausgewählt sind aus der Gruppe, bestehend aus Alkyl, Cycloalkyl, Alkenyl, Alkinyl, Aryl, Alkoxyalkyl und Aminoalkyl.

Besonders bevorzugt werden in dem erfindungsgemäßen Verfahren Verbindungen hydriert, die ausgewählt sind aus der Gruppe, bestehend aus Dimethylaminopropionitril, Aminoacetonitril, Formaldehydcyanhydrin, 3-(2-)Ethylhexoyl(proprionitril), 3-Dimethylaminoproprionitril, Methoxyproprionitril und Fettsäurenitrile. Besonders bevorzugt sind darüber hinaus Adipodinitril, Iminodiacetonnitril, Isophoronnitrilimin, Korksäuredinitril, Nitrilotriacetonitril und Isophthalsäuredinitril.

Die durch das erfindungsgemäße Verfahren erhaltenen Amine besitzen entsprechend die allgemeinen Strukturformeln

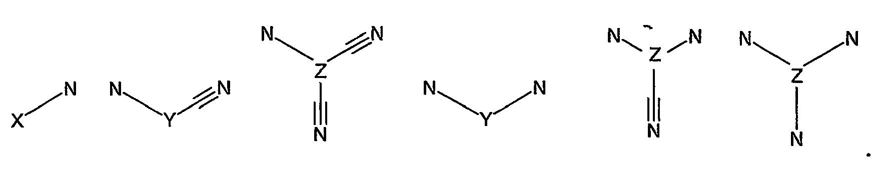

Die durch das erfindungsgemäße Verfahren hergestellten Verbindungen sind an sich bekannt. Insbesondere sind hierbei zu nennen Aminocapronitril, Hexamethylendiamin, Diethylentriamin, Ethylendiamin, Ethanolamin, Aminoacetonitril, Trisaminoethylamin, Dimethylaminopropanamin, Methoxypropylamin, 3-(2-Ethoxyhexoyl)propylamin, Isophorondiamin, 8-Aminooctanitril, Diaminooctan und Fettsäureamine.

Die Herstellung der in dem erfindungsgemäßen Verfahren als Edukte verwendeten Nitrile kann durch jede dem Fachmann bekannte Verfahrensweise erfolgen. Beispiele hierfür sind die Kolbe-Nitril-Synthese, die Strecker-Synthese, das Erhitzen von Säureamiden mit wasserabspaltenden Mitteln, die Addition an ungesättigte Nitrile und das Erhitzen von Aldoximen mit Acetanhydrid unter Wasserabspaltung.

Weiterer Gegenstand der vorliegenden Erfindung ist die Verwendung der zuvor beschriebenen ionischen Flüssigkeiten in Hydrierungen von in organischen Verbindungen enthaltenen Nitrilfunktionen an mindestens einem heterogenen Katalysator.

Bezüglich der organischen Verbindungen, die Nitrilfunktionen enthalten, den ionischen Flüssigkeiten und den heterogenen Katalysatoren wird auf obige Ausführungen verwiesen.

Das erfindungsgemäße Verfahren weist darüber hinaus noch nicht diskutierte Vorteile auf.

Das vorliegende Verfahren bietet beispielsweise die Möglichkeit, Produkte über eine Säure-Base-Reaktion mit den sauren ionischen Flüssigkeiten abzutrennen. Hierbei wird das Wertprodukt durch Destillation unter Verschiebung der Säure-Base-Gleichgewichte und Zersetzung der Anlagerungsverbindung ionische Flüssigkeit - Produkt abgetrennt. Damit entfällt eine teure und aufwendige Feststoffhandhabung durch Salzbildung bzw. - freisetzung.

Das beschriebene Verfahren führt, wie bereits ausgeführt, zu einer Erhöhung der Selektivität bzw. zu Beeinflussung des Produktverhältnisses zwischen Mono- und Dihydrierung bei Dinitrilen. Darüber hinaus sind die Ausbeute an Wertprodukt verbessert und die Lebensdauer des Katalysators erhöht. Die ionischen Flüssigkeiten lassen sich dabei durch Variation der verwendeten Kationen bzw. Anionen über einen weiten Bereich in Polarität, Löslichkeit, Benetzungsverhalten, Schmelzpunkt, physikalischen und chemischen Eigenschaften auf die Anforderungen maßschneidern.

Neben der erhöhten Selektivität ist ein weiterer bedeutender Vorteil der Erfindung, dass sie unter vergleichsweise niedrigem Druck und bei niedrigen Temperaturen ohne Ammoniak-Zusatz oder Zusatz anderer Additive vonstatten geht.

Daher sind aufwendige und teure Abfallbeseitigungen, z.B. durch Verbrennung von Additiven, hinfällig, ebenso wie entsprechende druckfest ausgelegte Apparate bzw. Verdichter zur Rückführung des in den bekannten Verfahren verwendeten Ammoniaks. Die verwendeten ionischen Flüssigkeiten sind im Gegensatz zu den meisten sonst verwendeten Additiven einschließlich Ammoniak durch den niedrigen Dampfdruck einfach und wirtschaftlich rückführbar.

Das erfindungsgemäße Verfahren wird anhand der folgenden Ausführungsbeispiele näher erläutert.

### Beispiele:

### Beispiel 1:

In einem Autoklaven werden Adipodinitril, Toluol, Ethylimidazoliumchlorid (Verhältnis Gew% 1:1:1) und Katalysator, bestehend aus Ru/Kohle (4,1 Gew%) gemischt und bei 100°C und einem Druck von 100 bar ohne Zusatz von Ammoniak umgesetzt. Nach 12 h wird die Reaktion durch Abkühlen abgebrochen, die Phasen getrennt, der Katalysator, der sich vor allem an der Phasengrenze ansammelt, abfiltriert und die beiden Phasen analysiert.
Umsatz: 60 %
Selektivität: 66 % bzgl. Aminocapronitril, 7 % bzgl. Hexamethylendiamin, Verhältnis Aminocapronitril zu Hexamethylendiamin: 5:1

### Beispiel 2:

In einem Autoklaven werden Adipodinitril, Toluol, Methylimidazoliumhydrogensulfat (Verhältnis Gew% 1:1:1) und Katalysator, bestehend aus Ru/Kohle (3 Gew%) gemischt und bei 100°C und 100 bar ohne Zusatz von Ammoniak umgesetzt. Nach 18 h wird die Reaktion durch Abkühlen abgebrochen, die Phasen getrennt, der Katalysator abfiltriert und die beiden Phasen analysiert.
Umsatz: 68 %
Selektivität: 53 % bzgl. Aminocapronitril, Verhältnis Aminocapronitril zu Hexamethylendiamin: 8:1

### Beispiel 3:

In einem Autoklaven werden Dimethylaminopropionitril, Toluol, Ethylimidazoliumchlorid im Verhältnis 1:1:1 und Kobaltkatalysator (4,1 Gew%) gemischt und bei 100°C und einem Wasserstoffdruck von 100 bar ohne Zusatz von Ammoniak umgesetzt. Nach 12 h wird die Reaktion durch Abkühlen abgebrochen, die Phasen getrennt, der Katalysator abfiltriert und die beiden Phasen analysiert.
Umsatz: 100 %
Selektivität: 90 % bzgl. Dimethylaminopropylamin

### Vergleichsbeispiel V1:

In einem Autoklaven werden Adipodinitril, Toluol (1:1 Gew%) und Katalysator, bestehend aus Ru/Kohle (5,6 Gew%), gemischt und bei 100°C und 100 bar ohne Zusatz von Ammoniak umgesetzt. Nach 12 h wird die Reaktion durch Abkühlen abgebrochen und der Reaktionsaustrag analysiert.
Umsatz: 100 %
Selektivität: 40 % bzgl. Hexamethylendiamin: Aminocapronitril und Adipodinitril sind nicht nachweisbar

### Vergleichsbeispiel V2:

In einem Autoklaven werden Adipodinitril, Toluol (1:1 Gew%) und Katalysator, bestehend aus Ru/Kohle (5,6 Gew%), gemischt und bei 100°C und 100 bar ohne Zusatz von Ammoniak umgesetzt. Nach 6 h wird eine Probe entnommen und der Reaktionsaustrag analysiert.
Umsatz: 69 %
Selektivität: 37 % bzgl. Aminocapronitril; Verhältnis Aminocapronitril zu Hexamethylendiamin: 3,2:1

## Patentansprüche

1. Verfahren zur Hydrierung von in organischen Verbindungen enthaltenen Nitrilfunktionen an mindestens einem heterogenen Katalysator, **dadurch gekennzeichnet, dass** die Hydrierung in Gegenwart einer ionischen Flüssigkeit durchgeführt wird und das Anion der ionischen Flüssigkeit ausgewählt ist aus der Gruppe, bestehend aus Halogenide F⁻, Cl⁻, Br⁻, I⁻, Acetat CH₃COO⁻, Trifluoracetat CF₃COO⁻, Triflat CF₃SO₃⁻, Sulfat SO₄²⁻, Hydrogensulfat HSO₄⁻, Methylsulfat CH₃OSO₃⁻, Ethylsulfat C₂H₅OSO₃⁻, Sulfit SO₃²⁻, Hydrogensulfit HSO₃⁻, Aluminiumchloride AlCl₄⁻, Al₂Cl₇⁻, Al₃Cl₁₀⁻, Aluminiumtetrabromid AlBr₄⁻, Nitrit NO₂⁻, Nitrat NO₃⁻, Kupferchlorid CuCl₂⁻, Phosphate, Phosphat PO₄³⁻, Hydrogenphosphat HPO₄²⁻, Dihydrogenphosphat H₂PO₄⁻, Carbonat CO₃²⁻, Hydrogencarbonat HCO₃⁻, Sulfonat - SO₃⁻, Tosylat p-CH₃C₆H₄SO₃⁻ und Bis(trifluormethylsulfonyl)imid (CF₃SO₂)₂N⁻ und die ionische Flüssigkeit Phosphoniumionen enthält und/oder mindestens einen fünf- oder sechsgliedrigen Heterocyclus enthält, der mindestens ein Phosphor- oder Stickstoffatom sowie gegebenenfalls ein Schwefel- und/oder Sauerstoffatom ausweist.

2. Verfahren nach Anspruch 1, **dadurch gekennzeichnet, dass** bei einem heterogenen Katalysator mit polarer Oberfläche eine unpolare ionische Flüssigkeit und bei einem heterogenen Katalysator mit einer unpolaren Oberfläche eine polare ionische Flüssigkeit verwendet werden und/oder ionische Flüssigkeit und Katalysator so gewählt werden, dass sich Edukt oder Produkt in einer anderen Phase aufhalten und/oder durch die ionische Flüssigkeit eine irreversible Belegung des Katalysators verhindert wird.

3. Verfahren nach Anspruch 1 oder 2, **dadurch gekennzeichnet, dass** die ionische Flüssigkeit einen Schmelzpunkt unterhalb 200°C aufweist

4. Verfahren nach einem der Ansprüche 1 bis 3, **dadurch gekennzeichnet, dass** das Verfahren in Abwesenheit vom Ammoniak durchgeführt wird.

5. Verfahren nach einem der Ansprüche 1 bis 4, **dadurch gekennzeichnet, dass** in Suspensionsfahrweise der Katalysator und/oder die ionische Flüssigkeit getrennt oder zusammen in das Verfahren zurückgeführt werden oder in einer Festbettfahrweise als ionische Flüssigkeit in das Verfahren zurückgeführt wird.

6. Verfahren nach einem der Ansprüche 1 bis 5, **dadurch gekennzeichnet, dass** ein heterogener Katalysator auf Basis von Nickel, Kobalt, Kupfer, Eisen, Ruthenium, Rhodium, Iridium, Palladium und/oder Platin, gegebenenfalls als Skelett-Katalysators, verwendet wird.

7. Verfahren nach einem der Ansprüche 1 bis 6, **dadurch gekennzeichnet, dass** die Hydrierung bei einer Temperatur von 20 bis 250°C und/oder einem Druck von 1 bis 300 bar durchgeführt wird.

8. Verfahren nach einem der Ansprüche 1 bis 7, **dadurch gekennzeichnet, dass** die zu hydrierenden Nitrile mindestens eine der folgenden Struktureinheiten aufweisen 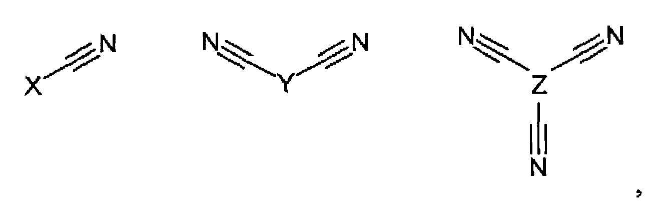 wobei in den Struktureinheiten X für lineare, verzweigte oder zyklische Gruppen steht, die ausgewählt sind aus der Gruppe bestehend aus Alkyl, Cycloalkyl, Alkenyl, Alkinyl, Aryl, Hydroxyalkyl, Alkoxyalkyl, Aminoalkyl und Aryl-C₁₋₄, Y und Z ausgewählt sind aus der Gruppe, bestehend aus Alkyl, Cycloalkyl, Alkenyl, Alkinyl, Aryl, Alkoxyalkyl und Aminoalkyl.

9. Verwendung von ionischen Flüssigkeiten in Hydrierungen von Nitrilfunktionen, die in organischen Verbindungen enthalten sind, an mindestens einem heterogenen Katalysator, wobei die Anionen der ionischen Flüssigkeit ausgewählt sind aus der Gruppe, bestehend aus Halogeniden F⁻, Cl⁻, Br⁻, I⁻, Acetat CH₃COO⁻, Trifluoracetat CF₃COO⁻, Triflat CF₃SO₃⁻, Sulfat SO₄²⁻, Hydrogensulfat HSO₄⁻, Methylsulfat CH₃OSO₃⁻, Ethylsulfat C₂H₅OSO₃⁻, Sulfit SO₃⁻, Hydrogensulfit HSO₃⁻, Aluminiumchloriden AlC₄⁻, Al₂Cl₇⁻, Al₃Cl₁₀⁻ ; Aluminiumtetrabromid AlBr₄⁻, Nitrit NO₂⁻, Nitrat NO₃⁻, Kupferchlorid CuCl₂⁻, Phosphaten, Phosphat PO₄³⁻, Hydrogenphosphat HPO₄²⁻, Dihydrogenphosphat H₂PO₄⁻, Carbonat CO₃²⁻, Hydrogencarbonat HCO₃⁻, Sulfonat -SO₃⁻, Tosylat p-CH₃C₆H₄SO₃⁻ und Bis(trifluormethylsulfonyl)imid (CF₃SO₂)₂N⁻ und die ionische Flüssigkeit Phosphoniumionen enthält und/oder mindestens einen fünf- oder sechsgliedrigen Heterocyclus enthält, der mindestens ein Phosphor- oder Stickstoffatom sowie gegebenenfalls ein Schwefel- und/oder Sauerstoffatom aufweist.

## Claims

1. A process for hydrogenating nitrile functions present in organic compounds over at least one heterogeneous catalyst, wherein the hydrogenation is carried out in the presence of an ionic liquid and the anion of the ionic liquid is selected from the group consisting of halides F⁻, Cl⁻, Br⁻, I⁻, acetate CH₃COO⁻, trifluoroacetate CF₃COO⁻, triflate CF₃SO₃⁻, sulfate SO₄²⁻, hydrogensulfate HSO₄⁻, methylsulfate CH₃OSO₃⁻, ethylsulfate C₂H₅OSO₃⁻, sulfite SO₃²-, hydrogensulfite HSO₃⁻, chloroaluminates AlCl₄⁻, Al₂Cl₇⁻, Al₃Cl₁₀⁻ , tetrabromoaluminate AlBr₄⁻, nitrite NO₂⁻, nitrate NO₃⁻, dichlorocuprate CuCl₂⁻, phosphates, phosphate PO₄³⁻, hydrogenphosphate HPO₄²⁻, dihydrogenphosphate H₂PO₄⁻, carbonate CO₃²⁻, hydrogencarbonate HCO₃⁻, sulfonate -SO₃⁻, tosylate p-CH₃C₆H₄SO₃⁻ and bis(trifluoromethylsulfonyl)imide (CF₃SO₂)₂N⁻ and the ionic liquid comprises phosphonium ions and/or at least one five- or six-membered heterocycle which has at least one phosphorus or nitrogen atom and, if appropriate, a sulfur and/or oxygen atom.

2. The process according to claim 1, wherein a nonpolar ionic liquid is used in the case of a heterogeneous catalyst having a polar surface and a polar ionic liquid is used in the case of a heterogeneous catalyst having a nonpolar surface and/or ionic liquid and catalyst are chosen so that starting material or product reside in a different phase and/or irreversible occupation of the catalyst is prevented by the ionic liquid.

3. The process according to claim 1 or 2, wherein the ionic liquid has a melting point below 200°C.

4. The process according to any of claims 1 to 3 carried out in the absence of ammonia.

5. The process according to any of claims 1 to 4, wherein, in the case of a suspension process, the catalyst and/or the ionic liquid are recirculated separately or together to the process or, in the case of a fixed-bed process, the ionic liquid is recirculated to the process.

6. The process according to any of claims 1 to 5, wherein a heterogeneous catalyst based on nickel, cobalt, copper, iron, ruthenium, rhodium, iridium, palladium and/or platinum is used, if appropriate as a skeletal catalyst.

7. The process according to any of claims 1 to 6, wherein the hydrogenation is carried out at a temperature of from 20 to 250°C and/or a pressure of from 1 to 300 bar.

8. The process according to any of claims 1 to 7, wherein the nitriles to be hydrogenated have at least one of the following structural units: 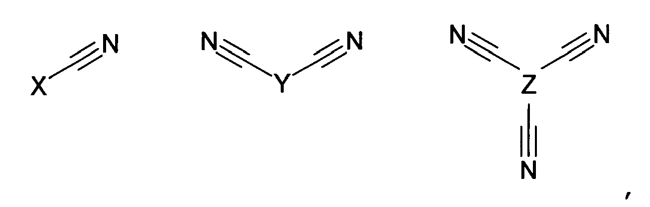 where X in the structural units is a linear, branched or cyclic group selected from the group consisting of alkyl, cycloalkyl, alkenyl, alkynyl, aryl, hydroxyalkyl, alkoxyalkyl, aminoalkyl and C₁₋₄-aryl, Y and Z are selected from the group consisting of alkyl, cycloalkyl, alkenyl, alkynyl, aryl, alkoxyalkyl and aminoalkyl.

9. The use of ionic liquids in hydrogenations of nitrile functions present in organic compounds over at least one heterogeneous catalyst, the anions of the ionic liquid being selected from the group consisting of halides F⁻, Cl⁻, Br⁻, I⁻, acetate CH₃COO-, trifluoroacetate CF₃COO⁻, triflate CF₃SO3⁻, sulfate SO₄²⁻, hydrogensulfate HSO₄⁻, methylsulfate CH₃OSO₃⁻, ethylsulfate C₂H₅OSO₃⁻, sulfite SO₃²-, hydrogensulfite HSO₃⁻, chloroaluminates AlCl₄⁻, Al₂Cl₇⁻, Al₃Cl₁₀⁻, tetrabromoaluminate AlBr₄⁻, nitrite NO₂⁻, nitrate NO₃⁻, dichlorocuprate CuCl₂⁻, phosphates, phosphate PO₄³⁻, hydrogenphosphate HPO₄²⁻, dihydrogenphosphate H₂PO₄⁻, carbonate CO₃²⁻, hydrogencarbonate HCO₃⁻, sulfonate -SO₃⁻, tosylate p-CH₃C₆H₄SO₃⁻ and bis(trifluoromethylsulfonyl)imide (CF₃SO₂)₂N⁻ and the ionic liquid comprising phosphonium ions and/or at least one five- or six-membered heterocycle which has at least one phosphorus or nitrogen atom and, if appropriate, a sulfur and/or oxygen atom.

## Revendications

1. Procédé d'hydrogénation de fonctions nitrile contenues dans des composés organiques sur au moins un catalyseur hétérogène, **caractérisé en ce que** l'hydrogénation est réalisée en présence d'un liquide ionique et l'anion du liquide ionique est choisi dans le groupe constitué par les anions halogénures F⁻, Cl⁻, Br⁻, I⁻, acétate CH₃COO⁻, trifluoroacétate CF₃COO⁻, triflate CF₃SO₃⁻, sulfate SO₄²⁻, hydrogénosulfate HSO₄⁻, méthylsulfate CH₃OSO₃⁻, éthylsulfate C₂H₅OSO₃⁻, sulfite SO₃²⁻, hydrogénosulfite HSO₃⁻, chlorures d'aluminium AlCl₄⁻, Al₂Cl₇⁻ , Al₃Cl₁₀⁻, tétrabromure d'aluminium AlBr₄⁻, nitrite NO₂⁻, nitrate NO₃⁻, chlorure de cuivre CuCl₂⁻, phosphates, phosphate PO₄³⁻, hydrogénophosphate HPO₄²⁻, dihydrogénophosphate H₂PO₄⁻, carbonate CO₃²⁻, hydrogénocarbonate HCO₃⁻, sulfonate -SO₃⁻, tosylate p-CH₃C₆H₄SO₃⁻ et bis (trifluorométhylsulfonyl) imide (CF₃SO₂)₂N⁻ et le liquide ionique contient des ions phosphonium et/ou au moins un hétérocycle de cinq ou six chaînons, qui présente au moins un atome de phosphore ou d'azote ainsi que le cas échéant un atome de soufre et/ou d'oxygène.

2. Procédé selon la revendication 1, **caractérisé en ce qu'**on utilise, dans le cas d'un catalyseur hétérogène avec une surface polaire, un liquide ionique non polaire et, dans le cas d'un catalyseur hétérogène avec une surface non polaire, un liquide ionique polaire et/ou le liquide ionique et le catalyseur sont choisis de manière telle que le produit de départ ou le produit restent dans une autre phase et/ou un recouvrement irréversible du catalyseur est empêché par le liquide ionique.

3. Procédé selon la revendication 1 ou 2, **caractérisé en ce que** le liquide ionique présente un point de fusion inférieur à 200°C.

4. Procédé selon l'une quelconque des revendications 1 à 3, **caractérisé en ce que** le procédé est réalisé en l'absence d'ammoniac.

5. Procédé selon l'une quelconque des revendications 1 à 4, **caractérisé en ce que** dans le mode opératoire en suspension, le catalyseur et/ou le liquide ionique sont recyclés séparément ou ensemble dans le procédé ou, dans le mode en lit fixe, le liquide ionique est recyclé dans le procédé.

6. Procédé selon l'une quelconque des revendications 1 à 5, **caractérisé en ce qu'**on utilise un catalyseur hétérogène à base de nickel, de cobalt, de cuivre, de fer, de ruthénium, de rhodium, d'iridium, de palladium et/ou de platine, le cas échéant comme catalyseur à squelette.

7. Procédé selon l'une quelconque des revendications 1 à 6, **caractérisé en ce que** l'hydrogénation est réalisée à une température de 20°C à 250°C et/ou à une pression de 1 bar à 300 bars.

8. Procédé selon l'une quelconque des revendications 1 à 7, **caractérisé en ce que** les nitriles à hydrogéner présentent au moins une des unités de structure suivantes 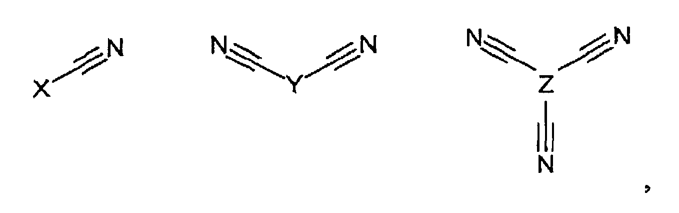 où, dans les unités de structure, X représente des groupes linéaires, ramifiés ou cycliques qui sont choisis dans le groupe constitué par alkyle, cycloalkyle, alcényle, alcynyle, aryle, hydroxyalkyle, alcoxyalkyle, aminoalkyle et aryle-C₁₋₄, Y et Z sont choisis dans le groupe constitué par alkyle, cycloalkyle, alcényle, alcynyle, aryle, alcoxyalkyle et aminoalkyle.

9. Utilisation de liquides ioniques dans des hydrogénations de fonctions nitrile qui sont contenues dans des composés organiques, sur au moins un catalyseur hétérogène, les anions du liquide ionique étant choisis dans le groupe constitué par les anions halogénures F⁻, Cl⁻, I⁻, acétate CH₃COO⁻, trifluoroacétate CF₃COO⁻, triflate CF₃SO₃⁻, sulfate SO₄²⁻, hydrogénosulfate HSO₄⁻, méthylsulfate CH₃OSO₃⁻, éthylsulfate C₂H₅OSO₃⁻, sulfite SO₃²⁻, hydrogénosulfite HSO₃⁻, chlorures d'aluminium AlCl₄⁻, Al₂Cl₇⁻, Al₃Cl₁₀⁻, tétrabromure d'aluminium AlBr₄⁻, nitrite NO₂⁻, nitrate NO₃⁻, chlorure de cuivre CuCl₂⁻, phosphates, phosphate PO₄³⁻, hydrogénophosphate HPO₄²⁻, dihydrogénophosphate H₂PO₄⁻, carbonate CO₃²⁻, hydrogénocarbonate HCO₃⁻, sulfonate -SO₃⁻, tosylate p-CH₃C₆H₄SO₃⁻ et bis (trifluorométhylsulfonyl) imide (CF₃SO₂)₂N⁻ et le liquide ionique contient des ions phosphonium et/ou au moins un hétérocycle à cinq ou six chaînons, qui présente au moins un atome de phosphore ou d'azote ainsi que le cas échéant un atome de soufre et/ou d'oxygène.
